# EUROPEAN PATENT APPLICATION

(11) **EP 1 055 429 A1**
(43) Date of publication of application: **29.11.2000**
(21) Application number: 99902905.1
(22) Date of filing: 15.02.1999
(51) Int. Cl.: A61K 39/09, A61K 38/16, C12N 15/09

(54) **NOVEL PREVENTIVES/REMEDIES FOR IMMUNOPATHY**

(30) Priority: 15.02.1998 JP 5013798
(71) Applicant: Juridical Foundation, The Chemo-Sero-Therapeutic Research Institute, Kumamoto-shi, Kumamoto 860-8568 (JP); KOWA COMPANY LTD., Nagoya-shi, Aichi-ken 460-0003 (JP)
(72) Inventor: SASAKI, Takumi, Kumamoto-shi, Kumamoto 862-8014 (JP); KIMACHI, Kazuhiko, Kumamoto-shi, Kumamoto 862-8002 (JP); SOEJIMA, Kenji, Kumamoto-shi, Kumamoto 862-8004 (JP); KIMURA, Yumi, Kikuchi, Kumamoto 869-1101 (JP); NOZAKI, Chikateru, Kumamoto-shi, Kumamoto 862-8001 (JP); FUJIYAMA, Yoshihide, Otsu-shi, Shiga 520-0865 (JP)
(74) Representative: Jaenichen, Hans-Rainer, Dr.
(86) International application number: JP9900638
(87) International publication number: WO9940935

(57) **Abstract**

A prophylactic/remedy for immunopathy for immunopathy comprising, as an active ingredient, modifications of Staphylococcal enterotoxin B (SEB) with substitution of at least one amino acid residues within the amino acid sequence of natural type SEB, or derivatives thereof, wherein said SEB modifications or derivatives thereof have inhibitory activity on T cell activation wherein they interact with specific Vβ component of T cell receptor (TCR) but are reduced in their immunological responsiveness to SEB without inducing elimination of T cells having specific Vβ component, the elimination being normally induced by natural type SEB or recombinant wild-type SEB.

## Description

### TECHNICAL FIELD OF THE INVENTION

The present invention relates to novel prophylactics/remedies for immunopathy. More specifically, the present invention relates to novel prophylactics/remedies for immunopathy such as rheumatoid arthritis, allergic diseases, etc. comprising as an active ingredient modifications of natural Staphylococcal enterotoxin B (hereinafter also referred to as "SEB"), known as one of superantigens, or derivatives thereof.

### BACKGROUND OF THE INVENTION

Autoimmune diseases are classified into two types: organ-nonspecific type autoimmune diseases such as rheumatoid arthritis (hereinafter also referred to as "RA") and organ-specific type autoimmune diseases such as ulcerative colitis. They are induced by T cells responsive to self antigens, said T cells being normally under immunological tolerance, that were activated within self tissues by some reasons to respond to self antigens, leading to continuous inflammatory reactions to thereby damage tissues. In such cases, self antigens are type II collagen that constitutes self joint or main components of the mucous membrane of the intestine, respectively.

The number of patients suffering from these diseases has slightly increased year by year but no effective remedies or prophylaxis have been found ("Immunodeficiency due to medicament", Men-eki Kagaku (Immunological Science), Vol. 9. p.285-289 (1984) Ed. by Yuichi Yamamura, Chuzo Kishimoto, Robert A. Good). Currently, for treatment of these diseases, there have been employed pharmacotherapy including administration of Salazopyrin, 5-aminosalycic acid, azathioprine, 6-MP, tranilast, methotrexate, cyclosporine A, or metronidazole, and administration of an excess amount of 7S-immunoglobulin; surgical therapy such as thymectomy or replacement with artificial joint; or symptomatic therapy such as nutritional therapy (Yoichi Ichikawa et al. "Study on efficacy of long-term administration of methotrexate and salazosulfapyridine on rheumatoid arthritis case" Rheumatism, Vol. 35, p.663-670, (1995); Sadao Kashiwazaki, "Study on efficacy of combination of auranofin and methotrexate on rheumatoid arthritis", Rheumatism, Vol. 36, p.528-544, (1996); Takefumi Furutani et al., "Detrimental event in therapy with low dose methotrexate on rheumatoid arthritis", Rheumatism, Vol. 36, p.746-752, (1996); Nobuo Watanabe, "Pharmacotherapy on juvenile rheumatoid arthritis", Rheumatism, Vol. 36, p.670-675, (1996); Takayasu Yakura, "Immunosuppressive therapy: Treatment of autoimmune diseases", Sogo Rinsho, Vol. 30, p.3358, (1981); Shin Totokawa et al., "Study on methotrexate therapy in rheumatoid arthritis: Seeking for strategy of more effective administration", Rheumatism, Vol. 37, p.681-687 (1997)). However, these therapies are not eradicative but rather are disadvantageous in that they may cause severe adverse side effects due to long-term ingestion of medicaments. Thus, it is desired to develop more effective prophylactics/remedies and therapy.

As is well known, SEB is one of bacterial superantigens (White J. et al., Cell, Vol. 56, p.27-35, 1989). Normal antigens, being complexed with class II Major Histocompatibility Complex (hereinafter also referred to as "MHC"), are recognized by T cell antigen receptor (hereinafter also referred to as "TCR"). This recognition is restricted to a haplotype of class II MHC, called "MHC restriction". On the contrary, superantigens are bound to class II MHC molecule irrespective of haplotype and further to specific β chain variable region (Vβ chain) of TCR. When such a linkage is formed, T cells bound with the superantigen are transiently activated, are promoted to divide and propagate and produce inflammatory cytokines (Micusan V.V. & Thibodean J., Seminars in Immunology, Vol. 5, p.3-11, 1993).

SEB is one of toxins causing food poisoning. Symptoms of food poisoning can be seen when SEB is intravenously administered to animals, suggesting that toxicity is exerted through the above-mentioned biological activity of SEB (Micusan V.V. & Thibodean J., Seminars in Immunology, Vol. 5, p.3-11, 1993).

However, when a superantigen is intravenously or intraperitoneally administered to newborn mice, a subpopulation of T cells having a Vβ chain responsive to the superantigen is eliminated so that said mice become non-responsive to said antigen, i.e. immunological tolerance (White J. et al., Cell, Vol. 56, p.27-35, 1989). On the other hand, when SEB is administered to adult mice, after transient activation as mentioned above, a subpopulation of T cells having responsive Vβ7, Vβ8 TCR becomes non-responsive to another stimulation with SEB, i.e. becomes anergy, to thereby cause immunological tolerance. With such activity, SEB is suggested to be potential prophylactics/remedies for intractable autoimmune diseases such as rheumatoid arthritis or ulcerative colitis or immunopathy (Japanese Patent Publication No. 9-110704). Since SEB is an enterotoxin causing staphylococcal food poisoning, pathogenicity of SEB is a problem when it is administered into the living body. In accordance with Japanese Patent Publication No. 9-110704, such toxicity is avoided by continuously administering a highly purified SEB in such a dose that does not render SEB being pathogenic through oral route for a long period of time to thereby effectively induce immunological tolerance without pathogenicity of SEB.

Another approach for avoiding SEB toxicity is "protective action of mutated superantigen" as disclosed by Kappler J. and Marrack P. et al. in Japanese Patent Publication No. 8-500328. Their invention is summarized as follows: SEB mutants are prepared and purified and those SEB mutants that could bind to both human class II MHC molecule and TCR were selected. Then, the SEB mutants, that bound to class II-positive cells to a degree undistinguishable from that of natural type SEB but did not stimulate propagation of T cells, were injected to an animal prior to exposition to SEB. As a result, the injected animal exhibited complete protection from toxicity of SEB.

The most important teaching of Japanese Patent Publication No. 8-500328 is a study on a structure of SEB that can be utilized for elimination of toxicity. A three-dimensional structure of SEB was reported by Swaminathan S. et al. (Nature, Vol. 359, p.801 (1992)). SEB molecule is consisted of two domains, i.e. the first domain consisted of residues 1 to 120 and the second consisted of residues 127-239. At the N-terminal of SEB, there have been identified three regions, i.e. Region 1 (residues 9 to 23), Region 2 (residues 41 to 53) and Region 3 (residues 60 to 61), that may affect class II MHC cell binding and/or TCR binding.

### DISCLOSURE OF THE INVENTION

Based on the above findings, the present inventors have completed the present invention that provides novel prophylactics/remedies for immunopathy comprising as an active ingredient SEB modifications that can effectively be used for prophylaxis and treatment of immunopathy with reduced toxicity or derivatives thereof.

That is, the present invention provides prophylactics/remedies for immunopathy comprising, as an active ingredient, modifications of Staphylococcal enterotoxin B (SEB) with substitution of at least one amino acid residues within the amino acid sequence of natural type SEB, or derivatives thereof, wherein said SEB modifications or derivatives thereof have inhibitory activity on T cell activation wherein they interact with specific Vβ component of T cell receptor (TCR) but are reduced in their immunological responsiveness to SEB without inducing elimination of T cells having specific Vβ component, the elimination being normally induced by natural type SEB or recombinant wild-type SEB.

In the first aspect of the present invention, the SEB modifications or derivatives thereof are SEB having amino acid substitution at the 9-position on the basis of natural type SEB with a protein-constituting amino acid other than aspartic acid, preferably asparagine, through site-specific mutagenesis, or derivatives thereof.

In the second aspect of the present invention, the SEB modifications or derivatives thereof are SEB having amino acid substitution at the 23-position on the basis of natural type SEB with a protein-constituting amino acid other than asparagine, preferably tyrosine, aspartic acid, isoleucine or lysine, through site-specific mutagenesis, or derivatives thereof.

In the third aspect of the present invention, the SEB modifications or derivatives thereof are SEB having amino acid substitution at the 41-position on the basis of natural type SEB with a protein-constituting amino acid other than isoleucine, preferably arginine or threonine, plus amino acid substitution either at the 44-position with a protein-constituting amino acid other than phenylalanine, preferably valine, or at the 45-position with a protein-constituting amino acid other than leucine, preferably valine, through site-specific mutagenesis, or derivatives thereof.

In the fourth aspect of the present invention, the SEB modifications or derivatives thereof are SEB having amino acid substitution at the 44-position on the basis of natural type SEB with a protein-constituting amino acid other than phenylalanine, preferably serine, through site-specific mutagenesis, or derivatives thereof.

### BRIEF DESCRIPTION OF THE DRAWINGS

Fig. 1 shows nucleotide sequences of each of PCR primers used for amino acid substitution for preparing each of SEB modifications of the present invention.
Fig. 2 shows nucleotide sequences of each of PCR primers used for amino acid substitution for preparing each of SEB modifications of the present invention.
Fig. 3 shows an amount of TNF-α produced by human peripheral blood mononuclear cells (PBMC) stimulated with wild-type SEB and SEB modifications.
Fig. 4 shows T cell inhibitory activity of SEB modifications.
Fig. 5 shows therapeutic effects of oral administration of SEB modifications on collagen-induced arthritis for effective cases wherein the axis of ordinate indicates severity of test mice. The axis of ordinates indicates severity of the tested mice and the axis of abscissas indicates days after initiation of administration of SEB modifications.
Fig. 6 shows therapeutic effects of oral administration of SEB modifications on collagen-induced arthritis for non-effective cases. The axis of ordinates indicates severity of the tested mice and the axis of abscissas indicates days after initiation of administration of SEB modifications.
Fig. 7 shows prophylactic effects of SEB modifications orally administered on collagen-induced arthritis. The axis of ordinate indicates severity of the tested mice and the axis of abscissas indicates days after initiation of administration of SEB modifications.

### BEST MODE FOR CARRYING OUT THE INVENTION

In accordance with the present invention, for preparing SEB modifications, those modifications that affect binding with class II MHC molecule were thoroughly investigated while there was introduced no modification at the sites involved in binding with TCR. Since linkage between superantigens and class II MHC molecule is indispensable to T cell activation by superantigens, it was expected that inhibition of SEB binding to class II MHC molecule would reduce responsiveness of T cells to SEB, i.e. anergy is induced without modification on TCR binding sites.

For Region 1 defined as amino acid residues Nos. 9 to 23, modifications of asparagine at the 23-position that block binding with class II MHC molecule were particularly investigated, including N23D, N23K, N23Y, and N23I. Modifications of aspartic acid at the 9-position such as D9N and of phenylalanine at the 17-position such as F17S were also investigated. The indication used herein for modifications is such that amino acids before and after modification are indicated before and after the number of position of amino acid residues where modifications occurred, respectively. For example, "N23D" means that "N" (Asn: asparagine) at the 23-position counted from the N-terminal is replaced with "D" (Asp: aspartic acid).

Region 2 defined as amino acid residue Nos. 40 to 53 is most important for mediating binding with class II MHC molecule in all the Staphylococcal enterotoxins. For Region 2, mutations at the 41-, 44-, 45-, and 53-positions were investigated. These amino acid residues are specific for binding to class II MHC molecule. Modifications were prepared by introducing point mutations through site-specific mutagenesis at the residues at the 41-, 44-, 45-, and 53-positions and in addition at the 59-position. Prepared modifications were: I41T L45V, I41R F44V, F44S, F44L I53N, and F44L I53N G59W.

The SEB modifications as mentioned above were estimated for their safety and effectiveness. As a result, it was found that the following embodiments of SEB modifications could be used as novel prophylactics/remedies for immunopathy such as rheumatoid arthritis with safe and excellent activities:
(1) SEB modifications having amino acid substitution at the 9-position on the basis of natural type SEB with a protein-constituting amino acid other than aspartic acid, particularly asparagine, or derivatives thereof;
(2) SEB modifications having amino acid substitution at the 23-position on the basis of natural type SEB with a protein-constituting amino acid other than asparagine, particularly tyrosine, aspartic acid, isoleucine or lysine, or derivatives thereof;
(3) SEB modifications having amino acid substitution at the 41-position on the basis of natural type SEB with a protein-constituting amino acid other than isoleucine, particularly arginine or threonine, plus amino acid substitution either at the 44-position with a protein-constituting amino acid other than phenylalanine, particularly valine, or at the 45-position with a protein-constituting amino acid other than leucine, particularly valine, or derivatives thereof; and
(4) SEB modifications having amino acid substitution at the 44-position on the basis of natural type SEB with a protein-constituting amino acid other than phenylalanine, particularly serine, or derivatives thereof.

It was also found that these SEB modifications could exert surprisingly excellent effects when they were administered through oral route.

First, reduced toxicity of the SEB modifications was investigated with induction of weight loss being as index. Weight loss was evaluated with BALB/c mice. A PBS (phosphate buffered saline) solution of 300 µg SEB, wherein endotoxin was removed up to a concentration that did not affect the experiment, was intraperitoneally administered to mice. Next day, weighing each mouse was started. As a result, wild type SEB and D9N showed weight loss by 7 to 9% until three days after administration whereas all the other SEB modifications did not show any weight loss.

Furthermore, the present inventors investigated these SEB modifications for their lethal toxicity induced by D-galactosamine. It was reported that administration of D-galactosamine induced drastic increase in sensitivity to SEB in mice, for example, LD50 of SEB was reduced to 2 µg/mice when D-galactosamine was administered to female BALB/c mice and 100% death was observed with administration of 20 µg/mice SEB (Miethke T. et al., J. Exp. Med., vol. 175, p.91-98 (1992)).

Using this experiment system, the present inventors investigated the SEB modifications for their lethal toxicity. As a result, wild type SEB and D9N showed lethality after 48 hours of 9/10 and 8/10, respectively, whereas I41T L45V showed 3/10, I41R F44V 1/10, F44S 0/10, F44L I53N 0/10, F44L I53N 1/10, and F44L I53N G59W 0/10, respectively. Thus, it was found that these SEB modifications had extremely reduced lethal toxicity. This expressly shows that the modifications according to the present invention not only can reduce toxicity as disclosed in Japanese Patent Publication No. 8-500328 but also eliminate lethal toxicity.

As described above, SEB is expected to be a potential prophylactic/remedy for immunopathy. However, it is also reported that SEB per se is involved in onset of immunopathy (Omata S. et al., Cellular Immunol., Vol. 179, p.138-145 (1997)). Thus, relationship between SEB per se as well as SEB modifications of the present invention and onset of immunopathy was investigated using collagen-induced arthritis (hereinafter also referred to as "CIA"), a model of human rheumatoid arthritis. Natural type SEB and SEB modifications provided by the present invention were administered to mice with arthritis induced by administration of collagen. In case of mice administered with natural type SEB, onset rate of arthritis was considerably high and severity of the disease was also significantly high, suggesting relationship between SEB and onset of the disease. On the contrary, onset of severe arthritis was not observed when SEB modifications were administered.

Some toxicity of SEB appears to be due to a monokine, especially tumor necrosis factor-α (TNF-α), produced by SEB-stimulated leukocytes. In fact, human peripheral blood lymphocytes produce a large amount of TNF-α when reacted with SEB. However, TNF-α level produced by SEB modifications was extremely low with stimulating activity of N23Y being the lowest.

As described above, SEB modifications exhibited lowered stimulating activity for propagation, cytokine production and the like. It is estimated that this lowered activity for stimulation is closely related to reduction of lethal toxicity in mice or loss of ability to induce arthritis. Interestingly, however, SEB modifications showed inhibitory activity to T lymphocyte activation although they had extremely reduced stimulating activity to T lymphocytes. When T cells are stimulated with antigens in the presence of antigen-presenting cells, it results in propagation of T cells or production by T cells of cytokines such as gamma-interferon (γ-IFN). However, addition of SEB modifications to this system inhibited these reactions. That is, it was found that SEB modifications not only exhibited reduced toxicity as compared to natural type SEB but also acquired additional activity to inhibit T cell activation, not shown in natural type SEB.

This inhibitory effect to T cell activation was also observed in vivo. In the above experimental system, when SEB modifications of the present invention were administered prior to administration of natural type SEB deeply involved in onset of arthritis, onset of severe arthritis could be protected, supporting excellent effects of administration of SEB modifications.

Whether SEB modifications of the present invention could be used as remedies for autoimmune disease through oral administration was investigated using collagen-induced arthritis (CIA) system. As a result, it was found that wild type and I41T L45V, I41R F44V, F44L I53N G59W, F44S, N23Y, N23I and N23D modifications inhibited exacerbation of arthritis to confirm effectiveness of these SEB modifications. Among these modifications, N23Y was the most potent inhibitor of CIA.

Accordingly, medicaments comprising SEB modifications in accordance with the present invention can be utilized as prophylactics/remedies for rheumatoid arthritis, particularly for oral administration, with lowered toxicity and more potent effectiveness based on inhibitory activity to T cell activation as compared to conventional medicaments. It is also expected that SEB modifications of the present invention exhibit prophylactic effects to inflammatory bowel diseases as in natural type SEB as disclosed in Japanese Patent Publication No. 9-110704.

The prophylactic/remedy for immunopathy for oral administration of the present invention is characterized by that it contains an extremely lowered amount of SEB modifications as compared to medicaments for another route of administration. These SEB modifications, like SEB, specifically induce weak tolerance to a subpopulation of T cells with SEB-binding Vβ TCR. On the other hand, they inhibit inflammatory activity of T cells when administered orally.

For preparing SEB modifications for use in the present invention, any method may be used without limitation. SEB modifications may be prepared, for example, by producing SEB-producing cells with the genetic engineering technique and separating therefrom SEB modifications produced.

For example, wild type SEB (i.e. SEB that is prepared with the genetic engineering technique and has the same amino acid sequence as that of SEB from Staphylococcus aureus), from which SEB modifications for use in the medicament of the present invention can be deduced, is prepared as outlined below.

Since chromosomal DNA of SEB is known (Ranelli D. M. et al., Proc. Natl. Acad. Sci. USA, Vol. 82, p.5850-(1985)), 5'-sense and 3'-antisense primers can be prepared with a DNA synthesizer etc. Plaque hybridization is carried out with these primers and chromosomal DNAs present in the commercially available SEB DNA library to give plaques. PCR is then carried out with the sense primer and DNAs are extracted from the formed bands. The extracted DNAs are then inserted into an appropriate cloning vector for cloning.

Cloned SEB-coding gene is cleaved with restriction enzymes such as SacI, HindIII, EcoRI, BamHI, XbaI, SalI and PstI and the obtained fragments are incorporated into a vector cleaved with restriction enzymes such as XmnI, HindIII, EcoRI, BamHI, XbaI, SalI and PstI to obtain recombinant DNA (Sambrook et al., Molecular Cloning, 2nd Ed., Chapter 9, 1989, New York, Cold Spring Harbor Laboratory Press). As a vector, expression vector for secretion pTrc99A etc. may suitably be employed.

The obtained recombinant DNA may be introduced into an appropriate host such as E. coli to give a transformant. After culturing said transformant in a usual manner, cells were separated after completion of culture. The separated cells were then ruptured in a usual manner and desired wild type SEB may be obtained from the suspension. Under suitable conditions, wild type SEB is conveniently secreted out into culture supernatant. In this case, the culture supernatant may be used as a starting material. A starting material is subjected to purification with a purification means such as, for example, immunoaffinity chromatography with absorbent to which anti-SEB monoclonal antibody is bound. A buffer used for final preparation in various experiments includes preferably Tris-HCl buffer, phosphate buffer, etc.

SEB modifications of the present invention may be prepared similarly as in preparation of wild type SEB as mentioned above provided that binding sites with class II MHC molecule is analyzed based on data of X-ray structural analysis of SEB, specific sites of the amino acid sequence to be modified is determined, appropriate primers are prepared and point mutations are introduced via PCR.

For maintaining the activity of wild type SEB or SEB modifications prepared, freshly prepared one is used or if stored at 4°C, it is preferably used within about five days after storage. Alternatively, SEB modifications of the present invention may be stored under suitable circumstances of gelatin, salts, sugars, sugar alcohols or amino acids etc.

In accordance with the present invention, SEB modifications or derivatives thereof may be combined with known suitable excipients and formulated into prophylactics/remedies for immunopathy of the present invention in the known manner. A dosage form of the medicament of the present invention may be any as far as it is suited for oral administration, such as in the form of powder (solid), solution or syrup. For example, in a preferable embodiment, SEB modifications or derivatives thereof are lyophilized together with a suitable excipient, for example, carbohydrates, sugars, sugar alcohols, or amino acids etc. to produce a solid preparation. In another preferable embodiment, SEB modifications or derivatives thereof are dissolved in physiological saline or in a suitable buffer with an ionic strength of acceptable tonicity to produce a liquid preparation. It is also conceivable that SEB modifications or derivatives thereof are dissolved in commercially available drink water and the solution is orally ingested. For a content in medicaments, SEB modifications or derivatives thereof are preferably present in an amount ranging from 0.05 µg to 50 mg (0.001 to 1,000 µg/kg body weight), preferably from 0.5 µg to 0.5 mg (0.01 to 10 µg/kg body weight) per administration.

An effective dose of prophylactics/remedies for immunopathy comprising as an active ingredient SEB modifications or derivatives thereof may vary depending on, for example, age of subject to be treated, symptoms or severity etc. and may finally be determined at physician's discretion. For example, the effective dose may typically be in a range of from 0.05 to 50,000 µg/day for adult calculated as SEB modifications, and preferably 0.5 to 500 µg in total may be orally administered once or twice a day. SEB modifications of the present invention may optionally be combined with other medicaments such as, for example, azathioprine, cyclophosphamide, or high molecular weight antiinflammatory agents such as anti-TNFα antibody.

Major immunopathy that can be cured with prophylactics/medicaments for immunopathy comprising SEB modifications of the present invention is autoimmune disease such as rheumatoid arthritis or ulcerative colitis. In addition to autoimmune disease, however, the prophylactics/medicaments for immunopathy of the present invention may also be applied to graft-versus-host disease after transplantation of bone marrow or type I, II, or III allergic diseases.

A "derivative" of SEB modifications of the present invention as used herein means the SEB modifications with substitution of at least one amino acid residues as mentioned above which are further modified. The present invention encompasses those SEB derivatives that have substitution, deletion or insertion at any amino acid residue within the amino acid sequence of natural type SEB in addition to the above specific amino acid residues in SEB modifications and still have an activity equivalent to that of SEB modifications.

### INDUSTRIAL APPLICABILITY

In accordance with the present invention, novel prophylactics/remedies for immunopathy including autoimmune diseases such as rheumatoid arthritis, provision of which having been earnestly desired over a long period of term, can be provided.

### EXAMPLE

The present invention is illustrated in more detail by means of the following Preparation and Examples, but should not be construed to be limited thereto.

### Preparation 1 (Preparation and expression of recombinant SEB modifications)

### 1-1 Cloning of SEB gene

A DNA library of Staphylococcus aureus enterotoxin A+B+D was purchased from CLONOTECH and plaque hybridization was carried out. As a probe, synthetic antisense DNA or PCR fragments were employed. A primer was added with SalI cleavage site at both ends for facilitating the subsequent cloning procedure.

Those plaques to which the primer bound were collected. PCR was carried out with a sense primer and DNA was extracted from the obtained bands and cloned into PCR-II vector (Invitrogen). The primers used in the above hybridization are depicted in Table 1.

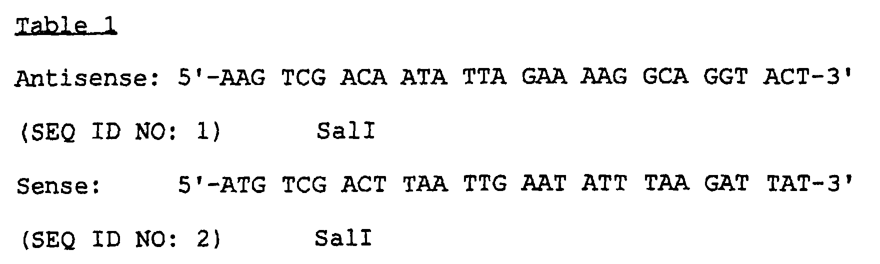

Subsequently, a nucleotide sequence of the DNA was determined with an automatic sequencer. The obtained SEB gene contained a promoter region (SEB-Pro). In order to obtain SEB gene without the promoter region, PCR was further conducted with primers listed in Table 2 and the obtained DNA fragments were cloned into PCR-II vector.

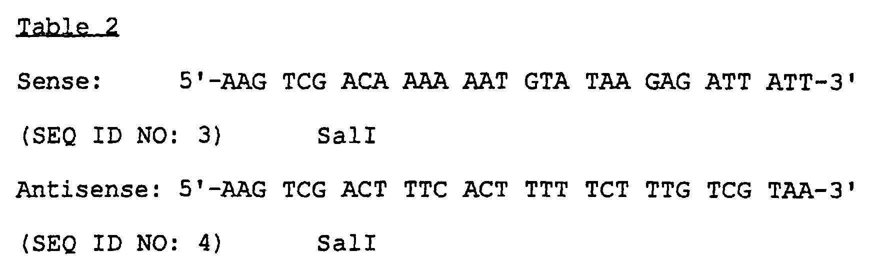

A nucleotide sequence of the DNA of the obtained SEB gene was determined with an automatic sequencer. It was found that the thus obtained SEB gene did not contain any mutation. The SEB gene without the promoter region was cleaved with SalI and cloned into an expression vector for secretion pTrc99A (Pharmacia Biotech) with the same cleavage site. Using the vector where the gene was inserted at the right direction, expression was induced with IPTG to confirm expression and secretion of SEB.

### 1-2 Polymerase chain reaction (PCR)

PCR was carried out with Taq polymerase and DNA thermal cycler of Perkin Elmer Cetus (Norwalk, CT, U.S.A.) as described in Saiki et al. (Science vol. 239, p.487 (1988)). Each cycle consisted of a denaturing process (94°C for 1 minute) for denaturing and releasing a duplicate template DNA, an annealing process (55°C for 2 minutes) for annealing the primer and the template, and an elongation process (72°C for 2 minutes) for synthesis. A total of 30 to 35 cycles were repeated. The template was present at a concentration of 1 to 10 M and the oligonucleotide primer at a concentration of 1 mM. A concentration of dNTP was 200 mM but was reduced to 20 mM for one dNTP when mutation is introduced.

### 1-3 Preparation and expression of recombinant SEB modification

Only SEB modifications with substitution of amino acid residues were prepared by the genetic recombination technique and expressed. SEB was modified for its region involved in binding to MHC class II molecule as taught by Marrack P. et al. (J. Exp. Med., Vol., 171, p.445 (1990)). Table 3 indicates sites where substitution of amino acid residues was introduced.

**Table 3**

| Sites to be modified | Alteration | Indication |
|---|---|---|
| D9 | Asn → Asp | D9N |
| F17 | Phe → Ser | F17S |
| N23 | Asn → Asp | N23D |
| N23 | Asn → Tyr | N23Y |
| N23 | Asn → Ile | N23I |
| N23 | Asn → Lys | N23K |
| D9, N23 | Asp → Asn | D9N N23D |
| | Asn → Asp | |
| F44 | Phe → Ser | F44S |
| F44, I53 | Phe → Leu | F44L I53N |
| | Ile → Asn | |
| F44, I53, G59 | Phe → Leu | F44L I53N G59W |
| | Ile → Asn | |
| | Gly → Trp | |
| I41, F44 | Ile → Arg | I41R F44V |
| | Phe → Val | |
| I41, L45 | Ile → Thr | I41T L45V |
| | Leu → Val | |

### 1-4 Introduction of amino acid substitution

The SEB-Pro gene inserted into PCRII vector was used as a template DNA and SfiI and NotI sites were introduced at the 5'- and 3,-ends, respectively, by PCR. The obtained DNA fragment was inserted into pTrc99ApelB vector and a nucleotide sequence was determined by sequencing. Each one primer at the sense and antisense sites for PCR were prepared bearing mutation within the nucleotide sequence so that natural amino acid residues at the desired sites are altered to desired amino acid residues for each of SEB modifications. The primers at the antisense site (D9N, N23I, N23Y, N23D, N23K, F17S, F17S N23D)-SEB were firstly employed for preparation. Then, PCR was conducted using a primer for introducing SfiI site as a sense primer.

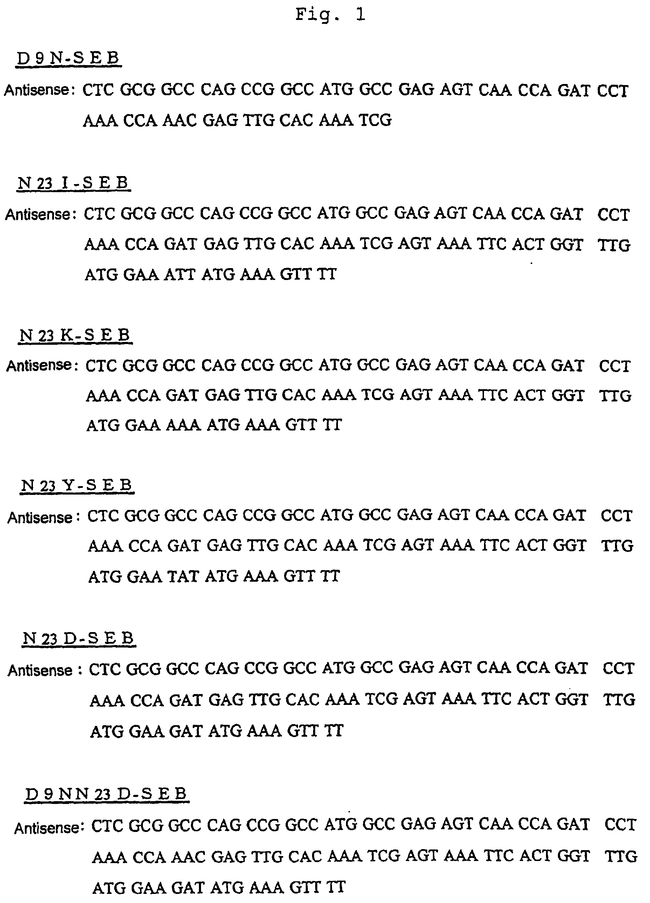

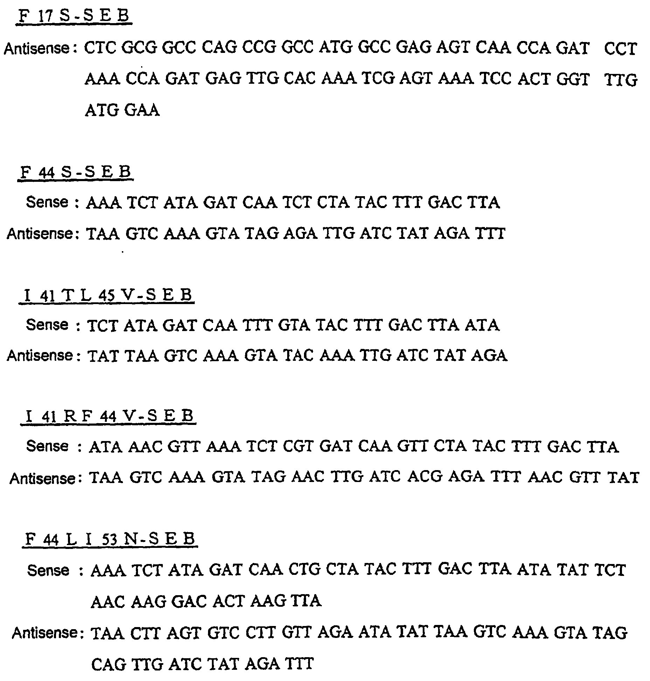

Figs. 1 and 2 show nucleotide sequences (SEQ ID NOs: 5 to 19) of PCR primers used for preparing SEB modifications.

Subsequently, PCR was carried out using a primer for introducing NotI site, prepared previously using a sense primer, as an antisense primer. PCR was then carried out using the obtained two DNA fragments. The obtained DNA fragments were cloned into pTrc99A. The vector was then treated with SfiI and NotI to investigate whether or not the vector was cleaved into DNA fragments with desired size. Those DNA fragments with desired size were sequenced for their nucleotide sequence to confirm that mutation was correctly introduced.

### 1-5 Expression of SEB modifications and preparation of said modifications

SEB modifications were expressed with modification genes inserted into pTrc99A vector. E. coli cells with the gene incorporated were cultured in culture medium in which 4% CIRCLEGROW (BIO IOI Inc., Vista, CA, U.S.A.) and ampicillin (50 mg/ml) were dissolved at 37°C for 18 hours. The cells were collected and then suspended in the same medium to 0.3-1.0 of O.D. 550 nm. Thereto was added 2 mM isopropyl-B-D(-)-thiogalactopyranoside (IPTG) and expression was induced by shaking the mixture at 37°C overnight. After induction, the host E. coli cells were removed by centrifugation and supernatant was filtered through 0.45 µm filter membrane.

The thus prepared supernatant was passed through Sepharose 4B column to which anti-SEB monoclonal antibody SA58-2-6IgG was immobilized so that SEB modifications in the supernatant were adsorbed. The column was washed with 0.1 M Tris HCl (pH 8.0) and then eluted with 4M MgCl₂. The eluted fractions were dialyzed against 20-fold volume of physiological saline three times and then against 20-fold volume of PBS twice. All the SEB modifications prepared herein could be purified with the above monoclonal antibody column.

### Example 1 (Lethal toxicity test with mice)

Natural type SEB does not provide mice with lethal toxicity. However, it is known that when D-galactosamine was previously administered, mice could be lead to death by intravenously or intraperitoneally administering 20 µg/mice of SEB as reported by Miethke T. et al. (J. Exp. Med. Vol., 175, p.91-98 (1992)). Thus, in order to investigate whether or not SEB modifications could reduce lethality, SEB or SEB modifications were administered to mice that previously received D-galactosamine.

Firstly, sensitivity to endotoxin was investigated. After administration of 20 mg/mice D-galactosamine to BALB/c mice, LPS (lipopolysaccharide) from E. coli B4 strain was intravenously administered to the mice and lethality after 24 hours was determined. As a result, death was not observed with the dose of not more than 1 ng/mice of LPS (Table 4).

**Table 4**

| Dose | Death No./Total No. |
|---|---|
| 1 µg/head | 7/9 |
| 100 ng/head | 8/9 |
| 10 g/head | 5/9 |
| 1 ng/head | 0/9 |
| 0.1 ng/head | 0/9 |

An endotoxin level in samples of SEB modifications was reduced so that a final dose becomes not more than 10 ng/mice and male mice were used for an experiment. In case of male mice, lethal toxicity of SEB after administration of D-galactosamine was not less than 100 µg/mice. Thus, SEB modifications were administered at a dose of 100 µg/mice. As shown in Table 5, natural type SEB, wild type SEB and D9N-SEB exhibited higher lethality, indicating lethal toxicity at that dose. In another SEB modifications, however, lethal toxicity was much reduced. "Natural type SEB" as used herein means enterotoxin from Staphylococcus aureus. "Wild type SEB" as used herein means SEB that has the same amino acid sequence as natural type SEB and is prepared by the genetic engineering technique.

**Table 5**

| SEB modifications (100 µg/head) | Lethality (total death No./ total No.) after: | |
|---|---|---|
| | 24 hours | 48 hours |
| Natural type | 8/10 | 8/10 |
| Wild type | 7/10 | 9/10 |
| D9N | 6/10 | 7/10 |
| I41T L45V | 3/10 | 3/10 |
| I41R F44V | 0/10 | 0/10 |
| F44L I53N | 0/9 | 0/9 |
| F44L I53N G59W | 0/10 | 0/10 |
| F44S | 0/10 | 0/10 |
| N23I | 0/10 | 0/10 |
| N23D | 0/10 | 0/10 |
| N23Y | 0/10 | 0/10 |
| N23K | 0/10 | 0/10 |
| PBS | 0/10 | 0/10 |

The same experiment was also carried out with female BALB/c mice. In case of female mice, high lethal toxicity was observed with 20 µg/mice SEB when 40 mg/mice D-galactosamine was administered. Higher lethality resulted in case of natural type SEB, wild type SEB and D9N-SEB. However, another SEB modifications exhibited lower lethality to demonstrate that lethal toxicity was reduced.

### Example 2 (Comparison of TNF-α production by human peripheral blood mononuclear cells (PBMC) after stimulation with SEB or SEB modifications)

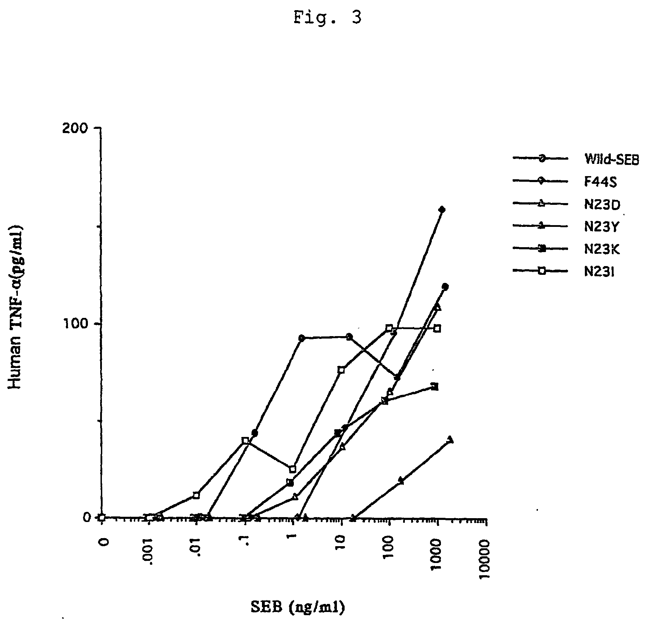

Peripheral blood mononuclear cells (PBMC) were collected from healthy adult using a lymphocyte separating reagent (Pharmacia) and suspended in RPMI1640 medium containing 10% FCS to make 1 × 10⁶ cells/ml. The cell suspension (100 ml) was added to 96-well microtiter plate (Falcon) and then a solution of SEB or SEB modifications (100 ml) was added and the plate was incubated at 37°C for 24 hours. After 24 hours, culture supernatant was collected and TNF-α in the supernatant was quantitated with an enzyme immunoassay kit for human TNF-α (Biosource). The results are shown in Fig. 3. When PBMC was stimulated with SEB, a large amount of TNF-α was produced. On the contrary, when PBMC was stimulated with SEB modifications, TNF-α production was drastically reduced. Among these, N23Y showed the lowest, i.e. one-thousandth or less of specific activity as compared to SEB.

### Example 3 (Inhibitory activity to T cells by SEB modifications)

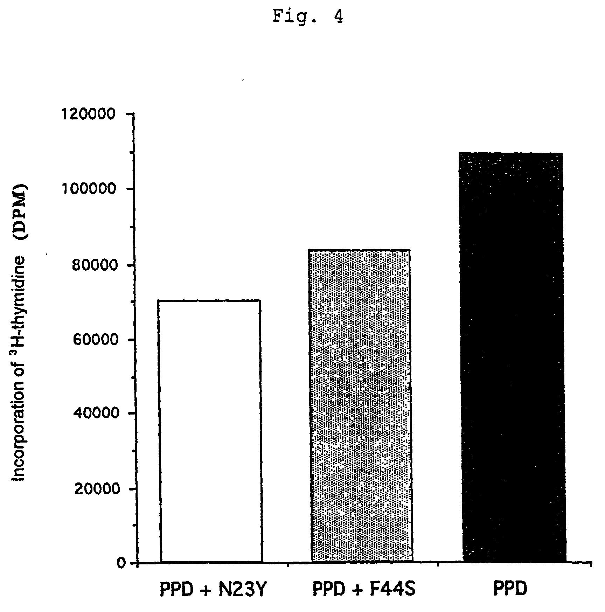

Inhibitory activity to T cells by SEB modifications was estimated with purified peptide derivative (PPD)-responsive mouse T cell line PPD914. This T cell line is Vβ8 TCR positive and can be responsive not only to its antigen PPD but also to SEB. 1 × 10⁴ PPD914 and 5 × 10⁵ mitomycin-treated spleen cells from DBA/1J were cultured on 96-well microtiter plate (Falcon) and stimulated with addition of 0.5 mg/ml PPD. SEB modifications (1 mg/ml) were added to this system and culture was continued at 37°C for 48 hours. After 48 hours, tritium thymidine was added at 0.5 mCi/well and culture was further continued for 16 hours and proliferation was determined. Inhibitory activity of SEB modifications was estimated by comparing with the results obtained in the absence of SEB modifications. The results are shown in Fig. 4. SEB modifications N23Y and F44S inhibited T cell proliferation induced by its antigen PPD.

### Example 4 (Estimation of SEB modifications for their safety in collagen-induced arthritis (CIA))

Collagen-induced arthritis (CIA) was induced in DBA/1 mice by intradermally administering 200 µg/mice of bovine type II collagen together with Freund's complete adjuvant to the mice. Twenty one days after administration of bovine type II collagen, each 50 µg/mice of SEB, F44S or N23D was intraperitoneally administered to the mice. A week later (28 days after the initial administration of collagen), onset rate of arthritis was compared. Table 6 summarizes the results of onset rate and severity of arthritis. Severe arthritis was observed in five among eight mice administered with SEB. On the contrary, no arthritis could be found in mice administered with F44S or N23D.

**Table 6**

| Primary immunization | Booster | Onset rate | Severity |
|---|---|---|---|
| Collagen | SEB | 5/8 | 6.2 |
| Collagen | F44S | 0/8 | - |
| Collagen | N23D | 0/8 | - |

### Example 5 (Prophylactic effect to SEB-induced arthritis by SEB modifications)

Each 50 µg/mice of F44S or N23D and phosphate buffer as control was intraperitoneally administered to the mice collagen-induced as in Example 4 eighteen days after administration of bovine type II collagen. Three days later, 50 µg/mice of SEB was intraperitoneally administered to the mice. As shown in Table 7, arthritis was observed in four among eight mice administered with phosphate buffer (onset rate of 50%). On the contrary, onset rate was reduced to 13% and 25% by previous administration of F44S or N23D, respectively.

**Table 7**

| Primary immunization | Pretreatment | Booster | Onset rate | Severity |
|---|---|---|---|---|
| Collagen | PBS | SEB | 4/8 | 6.5 |
| Collagen | F44S | SEB | 1/8 | 5.0 |
| Collagen | N23D | SEB | 2/8 | 2.5 |

### Example 6 (Curative effect of SEB modifications via oral administration on collagen-induced arthritis (CIA))

DBA/1 mice were subjected to CIA induction as in Example 4 and 21 days later were boostered with intraperitoneal administration of bovine collagen II. Onset of CIA was observed by measuring arthritis score. The severity of CIA was compared and the severity of the disease at the initiation of the experiment was made uniform. For oral administration, an amount of water taken by each mouse per day was determined and an average thereof was calculated. Mice were divided into groups so that the average amount of water taken by mice in each group may appropriately be diverted.

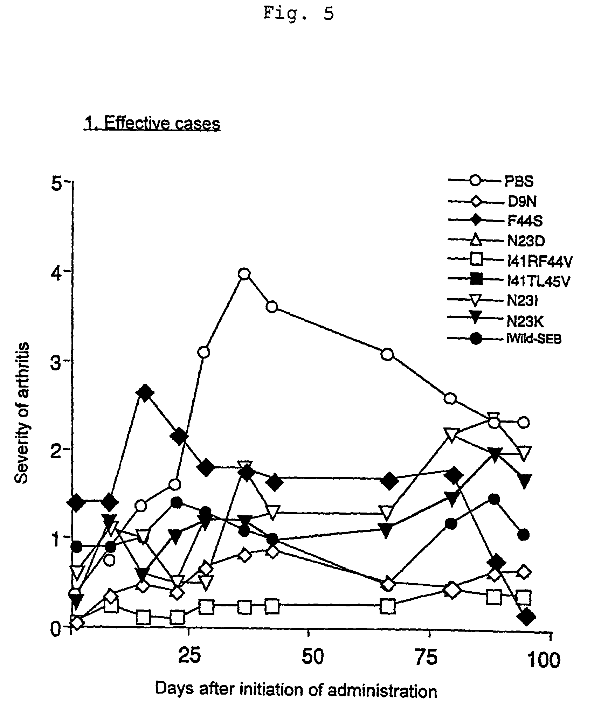

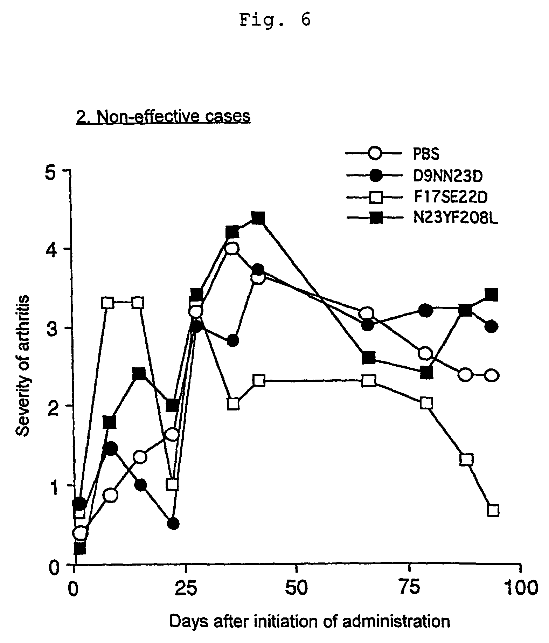

After division into groups, an experiment started wherein mice were allowed to drink water unrestrictedly to thereby take SEB modifications and reduction of disease was observed. The disease of each mouse was scored once a week and a change in the severity was recorded. These results are shown in Figs. 5 and 6. As to reduction of CIA symptoms and inhibitory activity to exacerbation of CIA, I41R F44V, I41T L45V, D9N, N23K, F44S, N23I and wild type SEB were shown to be effective. Based on arthritis score in each experimental group, a test of significance was conducted with Wilcoxon test between the groups administered with PBS (the group with onset of CIA) and with SEB modifications. As a result, it was found that all the above SEB modifications were significant with a risk of less than 0.05 during 25 to 75 days after the onset of disease and hence could alleviate the symptoms of CIA. Thus, efficacy of SEB modifications that have lowered toxicity and retain efficacy to CIA was demonstrated, suggesting that these SEB modifications would be a potential medicament for human rheumatoid arthritis for oral administration with much lowered adverse side effects.

On the other hand, D9N N23D, F17S E22D and N23Y F208L were not significant with a risk of 0.05 in Wilcoxon test in comparison with the PBS-administered group.

### Example 7 (Prophylactic effect of SEB modifications via oral administration on collagen-induced arthritis (CIA))

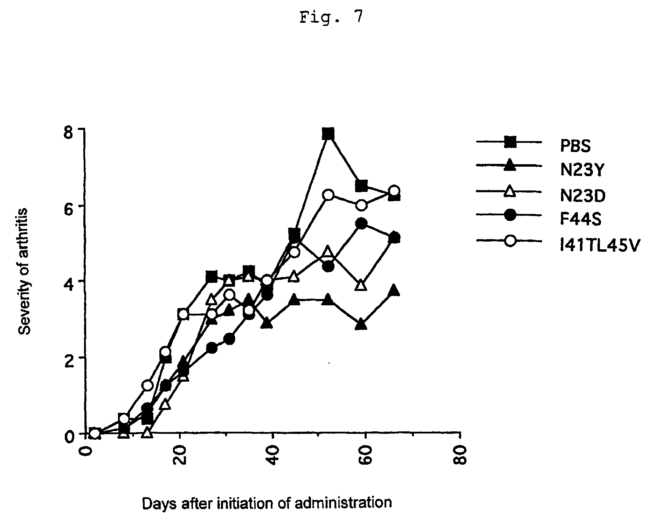

As described in Example 4, 200 µg of bovine type II collagen suspended in CFA was intradermally administered to DBA/I mice at the tail for immunization. Twenty days after the immunization, each 0.5 mg/mouse of SEB modifications dissolved in physiological saline was orally administered to mice every other day. On Day 30 after primary immunization, 100 µg of collagen was intraperitoneally administered (booster). Arthritis was observed with the lapse of time and scored to estimate CIA inhibitory activity of SEB modifications. As shown in Fig. 7, SEB modifications inhibited CIA even when they were administered 10 days prior to booster to demonstrate prophylactic effects of SEB modifications on arthritis. Among these SEB modifications, N23Y was the best and could alleviate arthritis remarkably.

## Claims

1. A prophylactic/remedy for immunopathy comprising, as an active ingredient, modifications of Staphylococcal enterotoxin B (SEB) with substitution of at least one amino acid residues within the amino acid sequence of natural type SEB, or derivatives thereof, wherein said SEB modifications or derivatives thereof have inhibitory activity on T cell activation wherein they interact with specific Vβ component of T cell receptor (TCR) but are reduced in their immunological responsiveness to SEB without inducing elimination of T cells having specific Vβ component, the elimination being normally induced by natural type SEB or recombinant wild-type SEB.

2. The prophylactic/remedy for immunopathy of claim 1 wherein said SEB modifications or derivatives thereof are SEB having amino acid substitution at the 9-position on the basis of natural type SEB with a protein-constituting amino acid other than aspartic acid, or derivatives thereof.

3. The prophylactic/remedy for immunopathy of claim 2 wherein said SEB modifications or derivatives thereof are SEB having amino acid substitution at the 9-position on the basis of natural type SEB with asparagine.

4. The prophylactic/remedy for immunopathy of claim 1 wherein said SEB modifications or derivatives thereof are SEB having amino acid substitution at the 23-position on the basis of natural type SEB with a protein-constituting amino acid other than asparagine, or derivatives thereof.

5. The prophylactic/remedy for immunopathy of claim 4 wherein said SEB modifications or derivatives thereof are SEB having amino acid substitution at the 23-position on the basis of natural type SEB with tyrosine, aspartic acid, isoleucine or lysine, or derivatives thereof.

6. The prophylactic/remedy for immunopathy of claim 1 wherein said SEB modifications or derivatives thereof are SEB having amino acid substitution at the 41-position on the basis of natural type SEB with a protein-constituting amino acid other than isoleucine plus amino acid substitution either at the 44-position with a protein-constituting amino acid other than phenylalanine or at the 45-position with a protein-constituting amino acid other than leucine, or derivatives thereof.

7. The prophylactic/remedy for immunopathy of claim 6 wherein said SEB modifications or derivatives thereof are SEB having amino acid substitution at the 41-position on the basis of natural type SEB with arginine or threonine plus amino acid substitution either at the 44-position with valine or at the 45-position with valine, or derivatives thereof.

8. The prophylactic/remedy for immunopathy of claim 1 wherein said SEB modifications or derivatives thereof are SEB having amino acid substitution at the 44-position on the basis of natural type SEB with a protein-constituting amino acid other than phenylalanine, or derivatives thereof.

9. The prophylactic/remedy for immunopathy of claim 8 wherein said SEB modifications or derivatives thereof are SEB having amino acid substitution at the 44-position on the basis of natural type SEB with serine, or derivatives thereof.

10. The prophylactic/remedy for immunopathy of claim 9 which is for oral administration.

11. The prophylactic/remedy for immunopathy of claim 10 which is an aqueous solution having pH and ionic strength that are physiologically acceptable.

12. The prophylactic/remedy for immunopathy of claim 11 wherein a substance selected from the group consisting of carbohydrates, sugars, sugar alcohols and amino acids is present in an amount sufficient for providing the solution with a physiologically acceptable tonicity.

13. The prophylactic/remedy for immunopathy of any one of claims 1 to 12 which is used for prophylactic/remedy of rheumatoid arthritis.
